Europäisches Patentamt

European Patent Office

Office européen des brevets

(11) Veröffentlichungsnummer: **0 353 503 B1**

(12) **EUROPÄISCHE PATENTSCHRIFT**

(45) Veröffentlichungstag der Patentschrift: **31.03.93**

(51) Int. Cl.5: **C07C 309/10**, C07C 303/02, C11D 1/16

(21) Anmeldenummer: **89112713.6**

(22) Anmeldetag: **12.07.89**

(54) **Kohlensäurefettalkoholester-sulfonate, Verfahren zu ihrer Herstellung und diese enthaltende oberflächenaktive Mittel.**

(30) Priorität: **20.07.88 DE 3824720**

(43) Veröffentlichungstag der Anmeldung:
**07.02.90 Patentblatt 90/06**

(45) Bekanntmachung des Hinweises auf die
Patenterteilung:
**31.03.93 Patentblatt 93/13**

(84) Benannte Vertragsstaaten:
**AT BE CH DE ES FR GB GR IT LI LU NL SE**

(56) Entgegenhaltungen:

**Keine Entgegenhaltungen**

(73) Patentinhaber: **Henkel Kommanditgesellschaft auf Aktien**
**Henkelstrasse 67**
**W-4000 Düsseldorf 13(DE)**

(72) Erfinder: **Fabry, Bernd, Dr.**
**Danziger Strasse 31**
**W-4052 Korschenbroich(DE)**
Erfinder: **Westfechtel, Alfred, Dr.**
**Kerschensteinerweg 9**
**W-4010 Hilden(DE)**
Erfinder: **Eierdanz, Horst, Dr.**
**Am Eichelkamp 116**
**W-4010 Hilden(DE)**
Erfinder: **Behler, Ansgar, Dr.**
**Siegfriedstrasse 80**
**W-4250 Bottrop(DE)**

Rank Xerox (UK) Business Services
(3.10/3.5x/3.0.1)

**Beschreibung**

Die Erfindung betrifft Kohlensäurefettalkoholester-sulfonate, erhältlich durch Sulfonierung von Kohlensäurefettalkoholestern der Formel I

$$R^1O\text{-}(C_mH_{2m}O)_n\text{-}CO\text{-}(OC_mH_{2m})_n\text{-}OR^2 \qquad (I)$$

in der

$R^1O$    einen Rest eines Fettalkohols mit 16 bis 22 Kohlenstoffatomen und mindestens einer olefinischen Doppelbindung,

$R^2O$    einen Rest eines gesättigten Alkanols mit 1 bis 22 Kohlenstoffatomen oder einen Rest eines Fettalkohols mit 16 bis 22 Kohlenstoffatomen und mindestens einer olefinischen Doppelbindung,

m    die Zahlen 2 und/oder 3 und

n    eine Zahl im Bereich von 0 bis 20

bedeuten sowie anschließender Neutralisation und Hydrolyse der Sulfonierungsprodukte mit Basen.

Sulfonate von Niedrigalkyl-Estern ungesättigter Carbonsäuren sind oberflächenaktive Verbindungen, die z.B. gemäß der EP-A 0 130 753 aus niedrigen Alkylestern ungesättigter Fettsäuren durch Umsetzung mit Schwefeltrioxid und anschließender Neutralisation sowie Hydrolyse hergestellt werden können. Die Struktur der letztlich erhaltenen Sulfonate ist noch nicht restlos aufgeklärt; es ist zu vermuten, daß dabei Gemische erhalten werden, die unter anderem aus Alken- und Hydroxyalkansulfonaten bestehen; vgl. J. Falbe, Surfactants in Consumer Products, p. 72-73, (1987), Springer-Verlag Berlin.

Es wurde nun gefunden, daß sich Kohlensäurefettalkoholestersulfonate, die Sulfonierungsprodukte der Kohlensäurefettalkoholester der obigen Formel I darstellen, Verbindungen mit interessanten oberflächenaktiven Eigenschaften sind, die zudem im pH-Bereich zwischen 0 und 13 überraschenderweise praktisch völlig hydrolysestabil sind.

Die Ausgangsprodukte für die Herstellung der Verbindungen der Erfindung, d.h. Fettsäureester der Kohlensäure, sind bekannt; sie lassen sich durch Umsetzung von Fettalkoholen mit Phosgen oder Chlorameisensäureester erhalten; vgl. J. Chem. Soc. 117, 708 (1920), J. Prakt. Chem. 22, 353-360 (1880). Eine noch einfachere Synthese beruht auf der Umesterung von Fettalkoholen mit Diethylcarbonat in Gegenwart alkalischer Katalysatoren; vgl. Chem. Ber. 114, 1210-1215 (1981), Houben/Weyl, Methoden der organischen Chemie, 4. Auflage, Bd. E4, S. 66 ff.. Analog lassen sich nach dem zuletztgenannten Verfahren Difettalkoholester oder gemischte Fettalkoholester der Kohlensäure herstellen.

In der allgemeinen Formel I bedeutet die Gruppe $R^1O$ einen Rest eines Fettalkohols mit 16 bis 22 Kohlenstoffatomen und einer olefinischen Doppelbindung oder mehreren, wobei eine olefinische Doppelbindung oder zwei bevorzugt sind. Derartige Fettalkohole können synthetischer und insbesondere natürlicher Herkunft sein. Typische Vertreter geeigneter natürlicher Fettalkohole mit einer oder zwei olefinischen Doppelbindungen sind Oleyl-, Elaidyl-, Linoleyl-, Gadoleyl-, Arachidon-, Eruca- und Brassidylalkohol. Wie in der Fettchemie üblich, können diese Alkohole in Form ihrer technischen Gemische mit anderen gesättigten oder ungesättigten Fettalkoholen eingesetzt werden, wie sie aus tierischen oder pflanzlichen Ölen und Fetten, z.B. Palmöl, Palmkernöl, Sojaöl, Rüböl, Olivenöl, Sonnenblumenöl und dergleichen durch Hydrierung der in diesen enthaltenen Fettsäureester zugänglich sind.

Im Rahmen der Erfindung besonders bevorzugt sind technische Cetyl/Oleyl-Schnitte mit Jodzahlen im Bereich von 50 bis 130.

Die Gruppe $R^2O$ kann die vorstehend für $R^1O$ genannte Bedeutung aufweisen; weiterhin kann die Gruppe $R^2O$ auch der Rest eines gesättigten Alkanols mit 1 bis 22 Kohlenstoffatomen sein. Typische Vertreter für derartige Alkanole sind Methanol, Ethanol, Propanol, Butanol, Pentanol, Hexanol, 2-Ethylhexanol, Octanol, Nonanol, Decanol, Dodecanol, Tetradecanol, Hexadecanol, Eicosanol und Docosanol. Besonders bevorzugt ist hier ein entweder von Ethanol oder von gesättigten Fettalkoholen mit 16 bis 18 Kohlenstoffatomen abgeleiteter Rest $R^2O$, wobei die gesättigten Fettalkohole, wie in der Fettchemie üblich, in Form ihrer an diesen Fettalkoholen reichen technischen Gemischen mit anderen Fettalkoholen eingesetzt werden.

Entsprechend der für die allgemeine Formel I angegebenen Bedeutung für m = 2 und/oder 3 können die Kohlensäurefettalkoholester auch in Form von Estern der Kohlensäure mit Anlagerungsprodukten von Ethylenoxid, Propylenoxid oder Ethylenoxid und Propylenoxid (in random- oder block-Verteilung der Propylenoxideinheiten) an ungesättigte Fettalkohole mit 16 bis 22 Kohlenstoffatomen bzw. gesättigte Alkanole mit 1 bis 22 Kohlenstoffatomen zum Einsatz kommen; jede der Gruppen $R^1O$ und $R^2O$ kann mit 1 bis 20 Ethylenoxy- und/oder Propylenoxy-Gruppen versehen sein.

Besonders bevorzugt sind Kohlensäurefettalkoholestersulfonate, die durch Sulfonierung von Kohlenfettalkoholestern der Formel I erhältlich sind, wobei

$R^1O$      einen Oleyloxyrest,

$R^2O$      einen Oleyloxy-, Cetyloxy-, Stearyloxy- oder Ethyloxyrest,

m      die Zahl 2 und

n      eine Zahl im Bereich von 0 bis 10

bedeuten.

Auch hier können Kohlensäurefettalkoholester eingesetzt werden, in denen die Gruppe $R^1O$ und gegebenenfalls die Gruppe $R^2O$ von technischen, oleylalkoholreichen Fettalkoholgemischen abgeleitet ist, wobei auch hier Anlagerungsprodukte von 1 bis 10 mol Ethylenoxid an die genannten Fettalkohole bzw. Fettalkoholgemische eingesetzt werden können.

Als Sulfonierungsmittel können die für die Sulfonierung von Olefinen üblichen eingesetzt werden, z.B. Schwefelsäure, Chlorsulfonsäure, Oleum, Amidosulfonsäure oder Schwefeltrioxid, wobei - insbesondere gasförmiges - Schwefeltrioxid bevorzugt ist.

Die erfindungsgemäß eingesetzten Kohlensäurefettalkoholester der Formel I weisen in der Gruppe $R^1O$ und gegebenenfalls auch in der Gruppe $R^2O$ je eine olefinische Doppelbindung auf; sie können bei der Herstellung der Titelverbindungen mit 1 mol Schwefeltrioxid pro Doppelbindung reagieren. Es ist jedoch nicht erforderlich, sämtliche olefinische Doppelbindungen der eingesetzten Kohlensäurefettakoholester zu sulfonieren, so daß man z.B. aus Kohlensäureestern, die zwei ungesättigte Fettalkoholreste enthalten, auch Kohlensäure-diesterderivate erhalten kann, die nur mit durchschnittlich 1 mol Schwefeltrioxid sulfoniert sind.

Die Sulfonierung von Kohlensäurefettalkoholestern der allgemeinen Formel I kann in der für Fettsäureniedrigalkylester bekannten Weise erfolgen, z.B. mit gasförmigem Schwefeltrioxid in hierfür geeigneten Reaktoren, insbesondere vom Typ der Fallfilmreaktoren. Dabei wird das Schwefeltrioxid mit Luft oder Stickstoff verdünnt und vorzugsweise in Form eines Gasgemisches mit ca. 1 bis 8, insbesondere 3 bis 5 Vol.-% Schwefeltrioxid eingesetzt. Bevorzugt wird die Reaktion in Abwesenheit von Lösemitteln durchgeführt; es können jedoch auch sämtliche für die Sulfonierung ungesättigter Fettsäureester, Olefine, Aromaten und dergleichen üblichen Lösemittel eingesetzt werden.

Die Herstellung der Kohlensäurefettalkoholester-sulfonate der Erfindung erfolgt bevorzugt durch Sulfonierung in einem Verhältnis von 0,5 bis 1,8, insbesondere 0,6 bis 1,5 und, besonders bevorzugt, von 1,0 bis 1,3 mol Schwefeltrioxid pro mol der in den Kohlensäurefettalkoholestern der allgemeinen Formel I enthaltenen olefinischen Doppelbindungen bei Temperaturen von 15 bis 80, insbesondere 40 bis 60°C.

Das dabei erhaltene Sulfonierungsprodukt wird anschließend mit Basen, insbesondere wässrigen Basen, unter Einhaltung eines pH-Wertes von mindestens 6 bis 13, insbesondere im Bereich zwischen 7 und 12, neutralisiert und hydrolysiert. Als Basen kommen hierbei Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak und organische Basen wie Ethanolamin, Diethanolamin oder Triethanolamin und primäre, sekundäre und tertiäre Alkylamine in Betracht. Alkalimetallund Erdalkalimetallhydroxide und Ammoniak kommen dabei vorzugsweise in Form mehr oder weniger konzentrierter wäßriger Lösungen zum Einsatz. Für die Neutralisation mit wasserfreien Basen eignen sich auch Alkali- und Erdalkalialkoxide.

Gemäß einer weiteren vorteilhaften Ausführungsform der Erfindung erfolgt die Hydrolyse der neutralisierten Sulfonierungsprodukte bei Temperaturen von mindestens 70°C innerhalb von 15 bis 240 Minuten, wobei die Obergrenze der Hydrolysetemperatur durch den Siedepunkt des Wassers bestimmt ist. Man kann jedoch auch bei Temperaturen über 100°C unter Druck hydrolysieren.

Wenn die eingesetzten Kohlensäurefettalkoholester, wie bei der Herstellung aus technischen Fettsäureschnitten durchaus möglich ist, Anteile an Estern gesättigter Fettalkohole enthalten, können diese in den Kohlensäurefettalkoholestersulfonaten der Erfindung verbleiben oder, falls erwünscht, in an sich bekannter Weise, z.B. durch Phasentrennung, abgetrennt werden. Entsprechende Verfahren sind an sich für Sulfonate von niedrig-Alkylestern ungesättigter Fettsäuren bekannt, vgl. die bereits genannte EP-A 0 130 753.

Bei der Herstellung der Kohlensäurefettalkoholester-sulfonate der Erfindung durch Sulfonierung von Kohlensäurefettalkoholestern der allgemeinen Formel I können sich intermediär cyclische bzw. verbrückte Sulfonierungsprodukte bilden, die eine Hydrolyse in der oben angegebenen Weise erforderlich machen, um ein allmähliches Nachsäuern der Produkte zu vermeiden. Dabei werden die enthaltenen Esterbindungen überraschenderweise nicht hydrolysiert.

Die Erfindung betrifft weiterhin ein Verfahren zur Herstellung von Kohlensäurefettalkoholester-sulfonaten mit den obengenannten Merkmalen. Weiterhin betrifft die Erfindung oberflächenaktive Mittel, die einzeln oder in Mischung Kohlensäurefettalkoholester-sulfonate der allgemeinen Formel II

$$R^3O\text{-}(C_mH_{2m}O)_n\text{-}CO\text{-}(OC_mH_{2m})_n\text{-}OR^4 \quad (II)$$

in der

R³O eine Gruppe der Formeln IIIa bzw. IIIb

$$CH_3-(CH_2)_x-CH(OH)-(CH_2)_y-CH(SO_3M)-(CH_2)_z-CH_2-O- \qquad (IIIa)$$

bzw.

$$CH_3-(CH_2)_x-CH(SO_3M)-(CH_2)_y-CH(OH)-(CH_2)_z-CH_2-O- \qquad (IIIb)$$

in welcher

$x$ und $z$ Zahlen im Bereich von 0 bis 18,

$y$ die Zahlen 0,1 oder 2 und

M ein Alkali-, Ammonium- oder gegebenenfalls mono-, di- oder trialkyl- bzw. hydroxyalkylsubstituiertes Ammoniumion ist,

wobei die Summe von $x + y + z$ eine Zahl im Bereich von 14 bis 18 ergibt, oder einen durch Abspaltung eines Moleküls Wasser aus der Gruppe der allgemeinen Formel IIIa bzw. IIIb gebildeter Rest ist, R⁴O eine Gruppe der allgemeinen Formel IIIa bzw. IIIb, in der $x$, $y$, $z$ und M wie oben definiert sind bzw. einen durch Abspaltung eines Moleküls Wasser aus der Gruppe der allgemeinen Formel IIIa bzw. IIIb gebildeteter Rest oder einen Rest eines Alkanols mit 1 bis 12 Kohlenstoffatomen,

$m$ die Zahlen 2 und/oder 3 und

$n$ eine Zahl im Bereich von 1 bis 20 bedeuten.

Bevorzugt sind oberflächenaktive Mittel, enthaltend - einzeln oder in Mischung - Kohlensäurefettalkoholestersulfonate der Formel II, in der

R³O einen Rest der Formel IIIa bzw. IIIb abgeleitet von einem Fettalkohol mit 18 Kohlenstoffatomen,

R⁴O einen Rest der Formel IIIa bzw. IIIb abgeleitet von einem Fettalkohol mit 18 Kohlenstoffatomen, eine geradkettige Alkoxygruppe mit 16 bis 18 Kohlenstoffatomen oder eine Ethoxygruppe,

$m$ die Zahl 2,

$n$ eine Zahl im Bereich von 0 bis 10,

$x$ und $z$ Zahlen im Bereich von 0 bis 14 und

$y$ eine Zahl im Bereich von 0, 1 oder 2

bedeuten,

wobei die Summe $x + y + z = 14$ und M wie oben definiert ist.

Typischerweise umfassen derartige Gemische oberflächenaktiver Mittel Verbindungen der allgemeinen Formel II, wobei R³O ein gegebenenfalls ethoxylierter und hydroxy-, sulfato-substituierter Oleyloxyrest ist. Die Gruppe R⁴O kann dabei die gleiche Bedeutung wie die für R³O angegebene aufweisen; in dem Gemisch sind jedoch auch Verbindungen vorhanden, bei denen R⁴O den Rest eines gesättigten Alkanols mit 1 bis 22 Kohlenstoffatomen bedeutet, z.B. eine Ethoxygruppe im Gemisch mit Cetyl- und/oder Stearyloxygruppen. Die Ethoxygruppe stammt dabei von bei der Herstellung der Ausgangsverbindung der Formel I nicht vollständig umgesetzten Diethylcarbonat, die Cetyloxy- oder Stearyloxygruppen stammen von in technischen Oleylalkoholschnitten vorhandenem Cetyl- und Stearylalkohol.

Die Erfindung wird im folgenden anhand bevorzugter Ausführungsbeispiele näher erläutert.

Herstellung von Ausgangsverbindungen.

A. Bisoleyl-carbonat.

Es wurde ein technischer Oleylalkohol mit den folgenden Kenndaten eingesetzt:

C-Kettenverteilung:

| | |
|---|---|
| $C_{14}$: | 0 bis 2 % |
| $C_{16}$: | 2 bis 10 % |
| $C_{18}$: | 87 bis 95 % |
| $C_{20}$: | 0 bis 3 % |
| Jodzahl: | 90 bis 97 |

OH-Zahl:     205 bis 215.

In einem 11-Rundkolben mit Destillationsaufsatz wurden 268 g (1 mol) des vorgenannten Oleylalkohols und 59 g (0,5 mol) Diethylcarbonat vorgelegt und mit 1 g (entsprechend 0,5 mol-%) Natriummethylat in Form einer 30 %-igen methanolischen Lösung versetzt. Die Reaktionsmischung wurde eine Stunde auf 120°C erhitzt und anschließend 30 min bei 150°C gehalten. Freigesetztes Ethanol wurde abdestilliert. Anschließend wurde das Produkt 2 h bei 120°C im Wasserstrahlvakuum von Restmengen Alkohol befreit und mit Essigsäure neutralisiert.

Man erhielt ca. 320 g Produkt als hellgelbe, leicht bewegliche Flüssigkeit mit einer OH-Zahl von 9,5, einer Säurezahl von 0,1, einer Jodzahl von 48 und einem Zahlenmittel des Molekulargewichts von 548.

B. Bis(oleyl-5EO)carbonat.

478 g (1 mol) eines handelsüblichen Anlagerungsproduktes von 5 mol Ethylenoxid an 1 mol des oben genannten Oleylalkohols wurden mit 59 g (0,5 mol) Diethylcarbonat in der unter A. beschriebenen Weise umgesetzt. Man erhielt die Titelverbindung in einer Ausbeute von ca. 550 g als hellgelbe, trübe Flüssigkeit mit einer OH-Zahl von 8,5, einer Säurezahl von 0,1, einer Jodzahl von 31 und einem Zahlenmittel des Molekulargewichts von 905.

C. Bis(oleyl-10EO)carbonat.

707 g (1 mol) eines handelsüblichen Anlagerungsproduktes von 10 mol Ethylenoxid an ein Mol des oben beschriebenen technischen Oleylalkohols wurden mit 59 g (0,5 mol) Diethylcarbonat in der unter A. beschriebenen Weise umgesetzt. Man erhielt die Titelverbindung in einer Ausbeute von ca. 760 g als schwach gelb gefärbte, trübe Flüssigkeit mit einer OH-Zahl von 7,3, einer Säurezahl von 0,1, einer Jodzahl von 20 und einem Zahlenmittel des Molekulargewichts von 1402.

D. Oleylcetylcarbonat.

Es wurde ein technisches Oleyl-/Cetylalkohol-Gemisch mit den folgenden Kenndaten eingesetzt:

C-Kettenverteilung:

| $C_{12}$ : | 0-2 % |
|---|---|
| $C_{14}$ : | 2-7 % |
| $C_{16}$ : | 27-35 % |
| $C_{18}$ : | 55-75 % |
| $C_{20}$ : | 0-2 % |
| Iodzahl: | 50-55 |
| OH-Zahl: | 210-220 |

260 g (1 mol) des technischen Oleyl-/Cetylalkohol-Gemisches wurden mit 59 g (0,5 mol) Diethylcarbonat in der unter A beschriebenen Weise umgesetzt.

Man erhielt die Titelverbindung in einer Ausbeute von ca. 310 g als farblose, feste Masse mit einer OH-Zahl von 9,0, einer Säurezahl von 0,1, einer Iodzahl von 24 und einem Zahlenmittel des Molekulargewichts von 548.

E. Oleylethylcarbonat

260 g (1 mol) eines technischen Oleylalkohols wurden mit 118 g (1 mol) Diethylcarbonat in der unter A beschriebenen Weise umgesetzt.

Man erhielt die Titelverbindung in einer Ausbeute von ca. 370 g als hellgelbe, klare Flüssigkeit mit einer OH-Zahl von 8,5, einer Säurezahl von 0,1, einer Iodzahl von 76 und einem Zahlenmittel des Molgewichts von 333.

Beispiel 1.

Bisoleyl-carbonat-disulfonat.

In einem 1l-Sulfierkolben mit Mantelkühlung und Gaseinleitungsrohr wurden 584 g (1 mol) Bisoleyl-carbonat vorgelegt und bei 40 bis 45°C mit 192 g (2,4 mol) gasförmigem Schwefeltrioxid umgesetzt.

Das Schwefeltrioxid wurde durch Erhitzen aus einer entsprechenden Menge 65 %-igen Oleums ausgetrieben, mit Stickstoff auf eine Konzentration von 5 Vol.-% verdünnt und innerhalb von 65 min in den Kohlensäurediester eingeleitet, wobei die Temperatur des Reaktionsgemisches durch Kühlung stets unter 60°C gehalten wurde.

Nach der Sulfonierung wurde das saure Reaktionsgemisch in eine Lösung von 104 g (2,6 mol) Natriumhydroxid in 500 ml Wasser eingerührt, 4 h bei 95°C hydrolysiert und anschliessend mit Mineralsäure auf einen pH-Wert von 7 gestellt.

| Kenndaten des Produkts: | |
|---|---|
| Aniontensid-Gehalt: | 16,4 % (0,0208 mval/g) |
| Unsulfierte Anteile: | 6,7 % |
| Sulfat (berechnet als $Na_2SO_4$): | 1,9 % |
| Wasser (nach Fischer): | 75,0 % |
| Klett-Farbzahl: | 102 |
| Zahlenmittel des Molgewichts: | 788 |

Hier wurden wie in den folgenden Beispielen Aniontensid-Gehalt und die unsulfierten Anteile nach den DGF-Einheitsmethoden, Stuttgart 1950 - 1984, H-III-10 bzw. G-III-6b ermittelt. Die Bestimmung der Klett-Farbzahl erfolgte nach 30 min Bleichung mit 5 Gew.-%-iger Wasserstoffperoxidlösung. Die Messung erfolgte bei einer Konzentration von 5 Gew.-% Aniontensid, bei pH 7, unter Verwendung einer 1 cm-Rundküvette und eines Blaufilters (400 bis 465 nm).

Beispiel 2.

Das Beispiel 1 wurde wiederholt, jedoch mit 224 g (2,8 mol) Schwefeltrioxid, entsprechend einem Verhältnis von Doppelbindungsäquivalenten : Schwefeltrioxid von 1:1,4. Die Einleitung des Schwefeltrioxids erfolgte innerhalb von 84 min; das rohe Sulfierprodukt wurde mit 120 g (3 mol) Natriumhydroxid in Wasser neutralisiert und anschließend auf einen pH-Wert von 6,5 bis 7,5 eingestellt.

| Kenndaten des Produkts: | |
|---|---|
| Aniontensid-Gehalt: | 24,1 % (0,0306 mval/g) |
| Unsulfierte Anteile: | 2,3 % |
| Sulfat (berechnet als $Na_2SO_4$): | 2,2 % |
| Wasser (nach Fischer): | 71,4 % |
| Klett-Farbzahl: | 286 |
| Zahlenmittel des Molgewichts: | 788 |

Beispiel 3.

Bis(oleyl-5EO)carbonat-disulfonat.

Analog zu der in Beispiel 1 beschriebenen Weise wurden 496 g des Anlagerungsprodukts von 5 mol Ethylenoxid an technischen Oleylalkohol mit 96 g (1,2 mol) Schwefeltrioxid entsprechend einem Verhältnis von Doppelbindungsäquivalenten zu Schwefeltrioxid von 1:1,4 umgesetzt.

| Kenndaten des Produkts: | |
|---|---|
| Aniontensid-Gehalt: | 16,8 % (0,0151 mval/g) |
| Unsulfierte Anteile: | 7,1 % |
| Sulfat (berechnet als $Na_2SO_4$): | 2,1 % |
| Wasser (nach Fischer): | 74,0 % |
| Klett-Farbzahl: | 96 |
| Zahlenmittel des Molgewichts: | 1108 |

Beispiel 4.

Bis(oleyl-10EO)carbonat-disulfonat.

Analog zu der in Beispiel 1 beschriebenen Weise wurden 701 g (0,5 mol) Bis(oleyl-10EO)carbonat mit 96 g (1,2 mol) Schwefeltrioxid entsprechend einem Verhältnis von Doppelbindungsäquivalenten zu Schwefeltrioxid von 1:1,4 umgesetzt.

| Kenndaten des Produkts: | |
|---|---|
| Aniontensid-Gehalt: | 19,0 % (0,0118 mval/g) |
| Unsulfierte Anteile: | 7,7 % |
| Sulfat (berechnet als $Na_2SO_4$): | 1,3 % |
| Wasser (nach Fischer): | 72,0 % |
| Klett-Farbzahl: | 41 |
| Zahlenmittel des Molgewichts: | 1606 |

Beispiel 5.

Oleylcetylcarbonat-sulfonat.

Analog zu der in Beispiel 1 beschriebenen Weise wurden 548 g (1 mol) Oleylcetylcarbonat mit 96 g (1,2 mol) Schwefeltrioxid entsprechend einem Verhältnis von Doppelbindungsäquivalenten : $SO_3$ von 1 : 1,2 umgesetzt und im Anschluß wie vorbeschrieben mit 56 g NaOH in Wasser neutralisiert.

| Kenndaten des Produkts: | |
|---|---|
| Aniontensid-Gehalt: | 9,8 % = 0,0150 mval/g |
| Unsulfierte Anteile: | 13,1 % |
| Sulfat (berechnet als $Na_2SO_4$): | 1,8 % |
| Wasser (nach Fischer): | 75,3 % |
| Klettfarbzahl: | 66 |
| Mittleres Molgewicht: | 650 |

Beispiel 6.

Oleylethylcarbonat-sulfat.

Analog zu der in Beispiel 1 beschriebenen Weise wurden 333 g (1 mol) Oleylethylcarbonat mit 96 g (1,2 mol) Schwefeltrioxid entsprechend einem Verhältnis von Doppelbindungsäquivalenten : $SO_3$ von 1 : 1,2 umgesetzt und im Anschluß wie vorbeschrieben mit 56 g NaOH in Wasser neutralisiert.

EP 0 353 503 B1

| Kenndaten des Produkts: | |
|---|---|
| Aniontensid-Gehalt: | 19,1 % = 0,0439 mval/g |
| Unsulfierte Anteile: | 7,5 % |
| Sulfat (berechnet als $Na_2SO_4$): | 1,4 % |
| Wasser (nach Fischer): | 72,0 % |
| Klettfarbzahl: | 96 |
| Mittleres Molgewicht: | 435 |

**Patentansprüche**

1. Kohlensäurefettalkoholester-sulfonate, erhältlich durch Sulfonierung von Kohlensäurefettalkoholestern der Formel I

$R^1O\text{-}(C_mH_{2m}O)_n\text{-}CO\text{-}(OC_mH_{2m})_n\text{-}OR^2$    (I)

in der
  $R^1O$    einen Rest eines Fettalkohols mit 16 bis 22 Kohlenstoffatomen und mindestens einer olefinischen Doppelbindung,
  $R^2O$    einen Rest eines gesättigten Alkanols mit 1 bis 22 Kohlenstoffatomen oder einen Rest eines Fettalkohols mit 16 bis 22 Kohlenstoffatomen und mindestens einer olefinischen Doppelbindung,
  m    die Zahlen 2 und/oder 3 und
  n    eine Zahl im Bereich von 0 bis 20
bedeuten,
sowie anschließender Neutralisation und Hydrolyse der Sulfonierungsprodukte mit Basen.

2. Kohlensäurefettalkoholester-sulfonate nach Anspruch 1, erhältlich durch Sulfonierung von Kohlensäurefettalkoholestern der allgemeinen Formel I, in der
  $R^1O$    einen Oleyloxyrest,
  $R^2O$    einen Oleyloxy-, Cetyloxy-, Stearyloxy- oder Ethyloxyrest,
  m    die Zahl 2 und
  n    eine Zahl im Bereich von 0 bis 10
bedeuten.

3. Kohlensäurefettalkoholester-sulfonate nach Anspruch 1 oder 2, erhältlich durch Sulfonierung von Kohlensäurefettalkoholestern der allgemeinen Formel I, in der $R^1O$, $R^2O$, m und n wie oben definiert sind, mit Schwefeltrioxid und anschließender Neutralisation und Hydrolyse der Sulfonierungsprodukte mit Basen.

4. Kohlensäurefettalkoholester-sulfonate nach mindestens einem der Ansprüche 1 bis 3, dadurch gekennzeichnet, daß die Sulfonierung, gegebenenfalls in inerten Lösemitteln, in einem Verhältnis von 0,5 bis 1,8, insbesondere 0,6 bis 1,5 mol Schwefeltrioxid pro mol der in den Kohlensäurefettalkoholestern der allgemeinen Formel I enthaltenen olefinischen Doppelbindungen bei Temperaturen von 15 bis 80, insbesondere 40 bis 60°C, erfolgt.

5. Kohlensäurefettalkoholester-sulfonate nach mindestens einem der Ansprüche 1 bis 4, dadurch gekennzeichnet, daß die Sulfonierung bei einem Verhältnis von 1,0 bis 1,3 Schwefeltrioxid pro mol olefinischer Doppelbindung erfolgt.

6. Kohlensäurefettalkoholester-sulfonate nach mindestens einem der Ansprüche 1 bis 5, dadurch gekennzeichnet, daß die Neutralisation und Hydrolyse der Sulfonierungsprodukte mit wässrigen Basen unter Einhaltung eines pH-Wertes von mindestens 6 bis 13, insbesondere 7 bis 12, erfolgt.

7. Kohlensäurefettalkoholester-sulfonate nach mindestens einem der Ansprüche 1 bis 6, dadurch gekennzeichnet, daß die Hydrolyse der neutralisierten Sulfonierungsprodukte bei Temperaturen von mindestens 70°C innerhalb von 15 bis 240 min erfolgt.

8

8. Verfahren zur Herstellung von Kohlensäurefettalkoholester-sulfonaten, dadurch gekennzeichnet, daß man Kohlensäurefettalkoholester der allgemeinen Formel I

$$R^1O-(C_mH_{2m}O)_n-O-CO-(OC_mH_{2m})_n-OR^2 \qquad (I)$$

in der

$R^1O$      einen Rest eines Fettalkohols mit 16 bis 22 Kohlenstoffatomen und mindestens einer olefinischen Doppelbindung,

$R^2O$      einen Rest eines gesättigten Alkanols mit 1 bis 22 Kohlenstoffatomen oder einen Rest eines Fettalkohols mit 16 bis 22 Kohlenstoffatomen und mindestens einer olefinischen Doppelbindung,

m      die Zahlen 2 und/oder 3 und

n      eine Zahl im Bereich von 0 bis 20

bedeuten,

sulfoniert und anschließend die Sulfonierungsprodukte mit Basen neutralisiert sowie hydrolysiert.

9. Verfahren nach Anspruch 8, dadurch gekennzeichnet, daß man Kohlensäurefettalkoholester der allgemeinen Formel I, in der

$R^1O$      einen Oleyloxyrest,

$R^2O$      einen Oleyloxy-, Cetyloxy-, Stearyloxy- oder Ethyloxyrest,

m      die Zahl 2 und

n      eine Zahl im Bereich von 0 bis 10

bedeuten,

sulfoniert.

10. Verfahren nach Anspruch 8 oder 9, dadurch gekennzeichnet, daß man die Sulfonierung von Kohlensäurefettalkoholestern der allgemeinen Formel I, in der $R^1O$, $R^2O$, m und n wie oben definiert sind, mit Schwefeltrioxid durchführt und anschließend mit Basen neutralisiert und hydrolysiert.

11. Verfahren nach mindestens einem der Ansprüche 8 bis 10, dadurch gekennzeichnet, daß die Sulfonierung, gegebenenfalls in inerten Lösemitteln, in einem Verhältnis von 0,5 bis 1,8, insbesondere 0,6 bis 1,5 mol Schwefeltrioxid pro mol der in den Kohlensäurefettalkoholestern der allgemeinen Formel I enthaltenen olefinischen Doppelbindungen bei Temperaturen von 15 bis 80, insbesondere 40 bis 60°C, erfolgt.

12. Verfahren nach mindestens einem der Ansprüche 8 bis 11, dadurch gekennzeichnet, daß die Sulfonierung bei einem Verhältnis von 1,0 bis 1,3 Schwefeltrioxid pro mol olefinischer Doppelbindung erfolgt.

13. Verfahren nach mindestens einem der Ansprüche 8 bis 12, dadurch gekennzeichnet, daß die Neutralisation und Hydrolyse der Sulfonierungsprodukte mit wässrigen Basen unter Einhaltung eines pH-Wertes von mindestens 6 bis 13, insbesondere 7 bis 12, erfolgt.

14. Verfahren nach mindestens einem der Ansprüche 8 bis 13, dadurch gekennzeichnet, daß die Hydrolyse der neutralisierten Sulfonierungsprodukte bei Temperaturen von mindestens 70°C innerhalb von 15 bis 240 min erfolgt.

15. Oberflächenaktive Mittel, enthaltend einzeln oder in Mischung Kohlensäurefettalkoholester-sulfonate der allgemeinen Formel II

$$R^3O-(C_mH_{2m}O)_n-CO-(OC_mH_{2m})_n-OR^4 \qquad (II)$$

in der $R^3O$ eine Gruppe der Formeln IIIa bzw. IIIb

$$CH_3-(CH_2)_x-CH(OH)-(CH_2)_y-CH(SO_3M)-(CH_2)_z-CH_2-O- \qquad (IIIa)$$

bzw.

$$CH_3-(CH_2)_x-CH(SO_3M)-(CH_2)_y-CH(OH)-(CH_2)_z-CH_2-O- \qquad (IIIb)$$

---

EP 0 353 503 B1

in welcher

x und *z*      Zahlen im Bereich von 0 bis 18,

y          die Zahlen 0,1 oder 2 und

M          ein Alkali-, Ammonium- oder gegebenenfalls mono-, di- oder trialkyl- bzw. hydroxyalkylsubstituiertes Ammoniumion ist,

wobei die Summe von x + y + z eine Zahl im Bereich von 14 bis 18 ergibt,

oder einen durch Abspaltung eines Moleküls Wasser aus der Gruppe der allgemeinen Formel IIIa bzw. IIIb gebildeter Rest ist,

$R^4O$      eine Gruppe der allgemeinen Formel IIIa bzw. IIIb, in der x, y, z und M wie oben definiert sind oder einen Rest eines Alkanols mit 1 bis 12 Kohlenstoffatomen,

m         die Zahlen 2 und/oder 3 und

n         eine Zahl im Bereich von 1 bis 20 bedeuten.

**16.** Oberflächenaktive Mittel nach Anspruch 15, enthaltend Kohlensäurefettalkoholester-sulfonate der allgemeinen Formeln II und IIIa bzw. IIIb, in denen

$R^3O$      einen Rest der Formel IIIa bzw. IIIb abgeleitet von einem Fettalkohol mit 18 Kohlenstoffatomen,

$R^4O$      einen Rest der Formel IIIa bzw. IIIb abgeleitet von einem Fettalkohol mit 18 Kohlenstoffatomen oder eine geradkettige Alkoxygruppe mit 16 bis 18 Kohlenstoffatomen,

m         die Zahl 2,

n         eine Zahl im Bereich von 0 bis 10,

x und z     Zahlen im Bereich von 0 bis 14 und

y         eine Zahl im Bereich von 0, 1 oder 2

bedeuten,

wobei die Summe x + y + z = 14 und M wie in Anspruch 15 definiert ist.

**17.** Verwendung von Kohlensäurefettalkoholester-sulfonaten nach Anspruch 1 oder 2 als oberflächenaktive Mittel.

## Claims

**1.** Carbonic acid fatty alcohol ester sulfonates obtainable by sulfonation of carbonic acid fatty alcohol esters corresponding to formula I

$$R^1O\text{-}(C_mH_{2m}O)_n\text{-}CO\text{-}(OC_mH_{2m})_n\text{-}OR^2 \qquad (I)$$

in which

$R^1O$      is a residue of a fatty alcohol containing 16 to 22 carbon atoms and at least one olefinic double bond,

$R^2O$      is a residue of a saturated alkanol containing 1 to 22 carbon atoms or a residue of a fatty alcohol containing 16 to 22 carbon atoms and at least one olefinic double bond,

m         is the number 2 and/or 3 and

n         is a number of 0 to 20,

and subsequent neutralization and hydrolysis of the sulfonation products with bases.

**2.** Carbonic acid fatty alcohol ester sulfonates as claimed in claim 1, obtainable by sulfonation of carbonic acid fatty alcohol esters corresponding to general formula I in which

$R^1O$      is an oleyloxy group,

$R^2O$      is an oleyloxy, cetyloxy, stearyloxy or ethyloxy group,

m         is the number 2 and

n         is a number of 0 to 10.

**3.** Carbonic acid fatty alcohol ester sulfonates as claimed in claim 1 or 2, obtainable by sulfonation of carbonic acid fatty alcohol esters corresponding to general formula I, in which $R^1O$, $R^2O$, m and n are as defined above, with sulfur trioxide and subsequent neutralization and hydrolysis of the sulfonation products with bases.

10

EP 0 353 503 B1

4. Carbonic acid fatty alcohol ester sulfonates as claimed in at least one of claims 1 to 3, characterized in that the sulfonation is carried out, optionally in inert solvents, in a ratio of 0.5 to 1.8 and more especially 0.6 to 1.5 mol sulfur trioxide per mol of the olefinic double bonds present in the carbonic acid fatty alcohol esters corresponding to general formula I at temperatures in the range from 15 to 80°C and more especially at temperatures in the range from 40 to 60°C.

5. Carbonic acid fatty alcohol ester sulfonates as claimed in at least one of claims 1 to 4, characterized in that the sulfonation is carried out in a ratio of 1.0 to 1.3 mol sulfur trioxide per mol olefinic double bond.

6. Carbonic acid fatty alcohol ester sulfonates as claimed in at least one of claims 1 to 5, characterized in that the neutralization and hydrolysis of the sulfonation products with aqueous bases is carried out at a pH value of at least 6 to 13 and more especially at a pH value of 7 to 12.

7. Carbonic acid fatty alcohol ester sulfonates as claimed in at least one of claims 1 to 6, characterized in that the hydrolysis of the neutralized sulfonation products is carried out for 15 to 240 minutes at temperatures of at least 70°C.

8. A process for the production of carbonic acid fatty alcohol ester sulfonates, characterized in that carbonic acid fatty alcohol esters corresponding to general formula I

$$R^1O\text{-}(C_mH_{2m}O)_n\text{-}CO\text{-}(OC_mH_{2m})_n\text{-}OR^2 \qquad (I)$$

in which
- $R^1O$     is a residue of a fatty alcohol containing 16 to 22 carbon atoms and at least one olefinic double bond,
- $R^2O$     is a residue of a saturated alkanol containing 1 to 22 carbon atoms or a residue of a fatty alcohol containing 16 to 22 carbon atoms and at least one olefinic double bond,
- m     is the number 2 and/or 3 and
- n     is a number of 0 to 20,

are sulfonated and the sulfonation products are subsequently neutralized and hydrolyzed with bases.

9. A process as claimed in claim 8, characterized in that carbonic acid fatty alcohol esters corresponding to general formula I in which
- $R^1O$     is an oleyloxy group,
- $R^2O$     is an oleyloxy, cetyloxy, stearyloxy or ethyloxy group,
- m     is the number 2 and
- n     is a number in the range from 0 to 10,

are sulfonated.

10. A process as claimed in claim 8 or 9, characterized in that the sulfonation of carbonic acid fatty alcohol esters corresponding to general formula I, in which $R^1O$, $R^2O$, m and n are as defined above, is carried out with sulfur trioxide, followed by neutralization and hydrolysis with bases.

11. A process as claimed in at least one of claims 8 to 10, characterized in that the sulfonation is carried out, optionally in inert solvents, in a ratio of 0.5 to 1.8 and more especially 0.6 to 1.5 mol sulfur trioxide per mol of the olefinic double bonds present in the carbonic acid fatty alcohol esters of general formula I at temperatures in the range from 15 to 80°C and more especially at temperatures in the range from 40 to 60°C.

12. A process as claimed in at least one of claims 8 to 11, characterized in that the sulfonation is carried out in a ratio of 1.0 to 1.3 mol sulfur trioxide per mol olefinic double bond.

13. A process as claimed in at least one of claims 8 to 12, characterized in that the sulfonation products are neutralized and hydrolyzed with aqueous bases at a pH value of at least 6 to 13 and more especially at a pH value of 7 to 12.

14. A process as claimed in at least one of claims 8 to 13, characterized in that the neutralized sulfonation products are hydrolyzed for 15 to 240 minutes at temperatures of at least 70°C.

11

15. Surface-active agents containing, individually or in admixture, carbonic acid fatty alcohol ester sulfonates corresponding to general formula II

$$R^3O-(C_mH_{2m}O)_n-CO-(OC_mH_{2m})_n-OR^4 \qquad (II)$$

in which

$R^3O$ is a group corresponding to formula IIIa or IIIb

$$CH_3-(CH_2)_x-CH(OH)-(CH_2)_y-CH(SO_3M)-(CH_2)_z-CH_2-O- \qquad (IIIa)$$

or

$$CH_3-(CH_2)_x-CH(SO_3M)-(CH_2)_y-CH(OH)-(CH_2)_z-CH_2-O- \qquad (IIIb)$$

in which

x and z are numbers of 0 to 18,
y is the number 0, 1 or 2 and
M is an alkali metal, ammonium or, optionally, mono-, di- or trialkyl- or hydroxyalkyl-substituted ammonium ion,
the sum of x + y + z being a number in the range from 14 to 18,
or a residue formed by elimination of one molecule of water from the group corresponding to general formula IIIa or IIIb,

$R^4O$ is a group corresponding to general formula IIIa or IIIb, in which x, y, z and M are as defined above, or a residue of an alkanol containing from 1 to 12 carbon atoms,
m is the number 2 and/or 3 and
n is a number in the range from 1 to 20.

16. Surface-active agents as claimed in claim 15 containing carbonic acid fatty alcohol ester sulfonates corresponding to general formulae II and IIIa or IIIb, in which

$R^3O$ is a residue of a $C_{18}$ fatty alcohol corresponding to formula IIIa or IIIb,
$R^4O$ is a residue of a $C_{18}$ fatty alcohol corresponding to formula IIIa or IIIb or is a linear $C_{16-18}$ alkoxy group,
m is the number 2,
n is a number of 0 to 10,
x and z are numbers of 0 to 14 and
y is the number 0, 1 or 2,
the sum x + y + z = 14 and M being as defined in claim 15.

17. The use of the carbonic acid fatty alcohol ester sulfonates claimed in claim 1 or 2 as surface-active agents.

## Revendications

1. Sulfonates d'esters d'alcools gras de l'acide carbonique, obtenables par sulfonation d'esters d'alcools gras de l'acide carbonique de la formule I

$$R^1O-(C_mH_{2m}O)_n-CO-(OC_mH_{2m})_n-OR^2 \qquad (I)$$

dans laquelle

$R^1O$ représente un radical d'un alcool gras comportant 16 à 22 atomes de carbone et au moins une double liaison oléfinique,
$R^2O$ correspond à un radical d'un alcanol saturé comportant 1 à 22 atomes de carbone ou à un radical d'un alcool gras comprenant renfermant 16 à 22 atomes de carbone et au moins une double liaison oléfinique,
m représente les nombres 2 et/ou 3 et
n est un nombre compris dans l'intervalle de 0 à 20,
ainsi que par neutralisation et hydrolyse ultérieures des produits de sulfonation à l'aide de bases.

12

**2.** Sulfonates d'esters d'alcools gras de l'acide carbonique selon la revendication 1, obtenables par sulfonation d'esters d'alcools gras de l'acide carbonique de la formule générale I, dans laquelle

$R^1O$ représente un radical oléyloxy,

$R^2O$ correspond à un radical oléyloxy, cétyloxy, stéaryloxy ou éthyloxy,

m représente le nombre 2

n est un nombre compris dans l'intervalle de 0 à 10.

**3.** Sulfonates d'esters d'alcools gras de l'acide carbonique selon la revendication 1 ou 2, obtenables par sulfonation d'esters d'alcools gras de l'acide carbonique de la formule générale I, dans laquelle $R^1O$, $R^2O$, m et n possèdent la valeur spécifiée ci-dessus, avec de l'anhydride sulfurique, suivie de neutralisation et d'hydrolyse des produits de sulfonation à l'aide de base.

**4.** Sulfonates d'esters d'alcools gras de l'acide carbonique selon au moins l'une des revendications 1 à 3, caractérisés en ce que la sulfonation se déroule, le cas échéant dans des solvants inertes, dans un rapport du 0,5 à 1,8, en particulier de 0,6 à 1,5 mole d'anhydride sulfurique par mole des doubles liaisons oléfiniques contenues dans les esters d'alcools gras de l'acide carbonique de la formule générale I, à des températures comprises entre 15 et 80 °C, en particulier entre 40 et 60 °C.

**5.** Sulfonates d'esters d'alcools gras de l'acide carbonique selon au moins l'une des revendications 1 à 4, caractérisés en ce que la sulfonation se déroule dans un rapport de 1,0 à 1,3 mole d'anhydride sulfonique par mole de double liaison oléfinique.

**6.** Sulfonates d'esters d'alcools gras de l'acide carbonique selon au moins l'une des revendications 1 à 5, caractérisés en ce que la neutralisation et l'hydrolyse des produits de sulfonation se déroule à l'aide de bases aqueuses, en respectant un pH d'au moins 6 à 13, en particulier de 7 à 12.

**7.** Sulfonates d'esters d'alcools gras de l'acide carbonique selon au moins l'une des revendications 1 à 6, caractérisés en ce que l'hydrolyse des produits de sulfonation neutralisés se déroule à des températures d'au moins 70 °C, dans un délai de 15 à 240 minutes.

**8.** Procédé de fabrication de sulfonates d'esters d'alcools gras de l'acide carbonique, caractérisé en ce que l'on sulfone des esters d'alcools gras de l'acide carbonique de la formule générale I

$$R^1O\text{-}(C_mH_{2m}O)_n\text{-}CO\text{-}(OC_mH_{2m})_n\text{-}OR^2 \qquad (I)$$

dans laquelle

$R^1O$ représente un radical d'un alcool gras comportant 16 à 22 atomes de carbone et au moins une double liaison oléfinique,

$R^2O$ correspond à un radical d'un alcanol saturé comportant 1 à 22 atomes de carbone ou à un radical d'un alcool gras comprenant renfermant 16 à 22 atomes de carbone et eu moins une double liaison oléfinique,

m représente les nombres 2 et/ou 3 et

n est un nombre compris dans l'intervalle de 0 à 20,

et en ce que l'on neutralise et hydrolyse ensuite les produits de sulfonation à l'aide de bases.

**9.** Procédé selon la revendication 8, caractérisé en ce que l'on sulfone des esters d'alcools gras de l'acide carbonique de la formule générale I, dans laquelle

$R^1O$ représente un radical oléyloxy,

$R^2O$ correspond à un radical oléyloxy, cétyloxy, stéaryloxy ou éthyloxy,

m représente le nombre 2

n est un nombre compris dans l'intervalle de 0 à 10.

**10.** Procédé selon la revendication 8 ou 9, caractérisé en ce que l'on effectue la sulfonation des esters d'alcools gras de l'acide carbonique de la formule générale I, dans laquelle $R^1O$, $R^2O$, m et n possèdent la valeur spécifiée ci-dessus, à l'aide d'anhydride sulfurique et que l'on fait suivre cette opération d'une neutralisation et d'une hydrolyse à l'aide de bases.

**11.** Procédé selon au moins l'une des revendications 8 à 10, caractérisé en ce que la sulfonation, le cas échéant dans des solvants inertes, se déroule dans un rapport de 0,5 à 1,8, en particulier de 0,6 à 1,5 mole d'anhydride sulfurique par mole des doubles liaisons oléfiniques contenues dans les esters d'alcools gras de l'acide carbonique de la formule générale I, à des températures comprises entre 15 et 80 ° C, en particulier entre 40 et 60 ° C.

**12.** Procédé selon au moins l'une des revendications 8 à 11, caractérisé en ce que la sulfonation se déroule dans un rapport de 1,0 à 1,3 mole d'anhydride sulfurique par mole de double liaison oléfinique.

**13.** Procédé selon au moins l'une des revendications 8 à 12, caractérisé en ce que la neutralisation et l'hydrolyse des produits de sulfonation se déroule à l'aide de bases aqueuses, en respectant un pH d'eu moins 6 à 13, en particulier de 7 à 12.

**14.** Procédé selon au moins l'une des revendications 8 à 13, caractérisé en ce que l'hydrolyse des produits de sulfonation neutralisés se déroule à des températures d'au moins 70 ° C, dans un délai de 15 à 240 minutes.

**15.** Agents tensioactifs, qui renferment, seuls ou en mélange, des sulfonates d'esters d'alcools gras de l'acide carbonique de la formule générale II

$$R^3O-(C_mH_{2m}O)_n-CO-(OC_mH_{2m})_n-OR^4 \qquad (II)$$

dans laquelle

$R^3O$ représente un groupe des formules IIIa ou IIIb

$$CH_3-(CH_2)_x-CH(OH)-(CH_2)_y-CH(SO_3M)-(CH_2)_z-CH_2-O- \qquad (IIIa)$$

ou

$$CH_3-(CH_2)_x-CH(SO_3M)-(CH_2)_y-CH(OH)-(CH_2)_z-CH_2-O- \qquad (IIIb)$$

dans laquelle

$x$ et $z$ correspondent à des nombres compris dans l'intervalle de 0 à 18,

$y$ représente les nombres 0, 1 ou 2 et

$M$ est un ion de métal alcalin, d'ammonium ou le cas échéant un ion d'ammonium à substitution mono-, di- ou trialkyle ou hydroxyalkyle,

la somme de $x + y + z$ étant égale à un nombre compris dans l'intervalle de 14 à 18, ou représente un radical formé par séparation d'une molécule d'eau, à partir du groupe de la formule générale IIIa ou IIIb,

$R^4O$ est un groupe de la formule générale IIIa ou IIIb, dans laquelle x, y, z et M possèdent la valeur indiquée ci-dessus ou un radical d'un alcanol comportant 1 à 12 atomes de carbone,

$m$ correspond aux nombres 2 et/ou 3 et

$n$ est un nombre compris dans l'intervalle de 1 à 20,

**16.** Agents tensioactifs selon la revendication 15, renfermant des sulfonates d'esters d'alcools gras de l'acide carbonique des formules générales II et IIIa ou IIIb, dans lesquelles

$R^3O$ représente un radical de la formule IIIa ou IIIb dérivé d'un alcool gras comportant 18 atomes de carbone,

$R^4O$ correspond à un radical de la formule IIIa ou IIIb dérivé d'un alcool gras comportant 18 atomes de carbone, ou à un groupe alcoxy à chaîne droite renfermant 16 à 18 atomes de carbone,

$m$ est le chiffre 2,

$n$ représente un nombre compris dans l'intervalle de 0 à 10,

$x$ et $z$ sont des nombres situé dans la plage de 0 à 14 et

$y$ est égal à 0, 1 ou 2,

la somme $x + y + z = 14$ et M possédant la signification mentionnée dans la revendication 15.

**17.** Utilisation des sulfonates d'esters d'alcools gras de l'acide carbonique selon la revendication 1 ou 2, comme agents tensioactifs.